# EUROPÄISCHE PATENTSCHRIFT

(11) **EP 0 513 099 B1**
(45) Veröffentlichungstag und Bekanntmachung des Hinweises auf die Patenterteilung: **13.10.1999**
(21) Anmeldenummer: 91903267.2
(22) Anmeldetag: 31.01.1991
(51) Int. Cl.: C09K 3/30, A61K 9/72

(54) **NEUE TREIBGASE UND IHRE VERWENDUNG IN ARZNEIMITTELZUBEREITUNGEN**
NOVEL VEHICLE GASES AND THEIR USE IN MEDICAL PREPARATIONS
NOUVEAUX GAZ PROPULSEURS ET LEUR UTILISATION DANS DES PREPARATIONS MEDICAMENTEUSES

(30) Priorität: 03.02.1990 DE 4003270
(43) Veröffentlichungstag der Anmeldung: 19.11.1992
(73) Patentinhaber: Boehringer Ingelheim Pharma KG, 55216 Ingelheim am Rhein (DE); BOEHRINGER INGELHEIM INTERNATIONAL GmbH, 55218 Ingelheim am Rhein (DE)
(72) Erfinder: WEIL, Hans-Hermann, D-6551 Gau-Bickelheim (DE); DAAB, Ottfried, D-6507 Ingelheim am Rhein (DE)
(74) Vertreter: Laudien, Dieter, Dr.
(86) Internationale Anmeldenummer: EP9100178
(87) Internationale Veröffentlichungsnummer: WO9111496

(56) Entgegenhaltungen:
- EP-A- 0 247 608
- EP-A- 0 384 371
- WO-A-86/04233
- World Patent Index, Derwent Publications Ltd, London & JP,A,55131096

## Beschreibung

Die Erfindung betrifft neue Arzneimittelsuspensionen in verflüssigten Treibgasen für die Erzeugung von Inhalationsaerosolen, in denen als typischer Bestandteil 1,1,1,2,3,3,3-Heptafluorpropan (TG 227) enthalten ist.

Aerosole von pulverförmigen (mikronisierten) Arzneistoffen werden vielfach in der Therapie, z.B. in der Therapie von obstruktiven Atemwegserkrankungen, eingesetzt. Soweit solche Aerosole nicht durch Zerstäuben des Arzneipulvers oder durch Versprühen von Lösungen erzeugt werden, benutzt man Suspensionen der Arzneistoffe in verflüssigten Treibgasen. Als solche dienen hauptsächlich Mischungen aus TG 11 (Trichlorfluormethan), TG 12 (Dichlordifluormethan) und TG 114 (1,2-Dichlor-1,1,2,2-tetrafluorethan), ggf. unter Zusatz von niederen Alkanen, etwa Butan, Pentan oder auch von DME (Dimethylether). Mischungen solcher Art sind beispielsweise aus der deutschen Patentschrift 1178975 bekannt.
Wegen ihres negativen Einflusses auf die Erdatmosphäre (Zerstörung der Ozonschicht, Treibhauseffekt) ist der Einsatz der Chlorfluorkohlenwasserstoffe zu einem Problem geworden, so daß nach anderen Treibgasen bzw. Treibgasmischungen gesucht wird, von denen die genannten negativen Wirkungen nicht oder wenigstens in geringerem Maß ausgehen.
Die Suche stößt jedoch auf erhebliche Schwierigkeiten, weil Treibgase, die therapeutisch eingesetzt werden sollen, zahlreiche Kriterien zu erfüllen haben, die nicht leicht in Einklang miteinander zu bringen sind, etwa hinsichtlich Toxizität, Stabilität, Dampfdruck, Dichte, Löseverhalten.

Aus der nachveröffentlichten EP-A-234 371 sind 1,1,1,2,3,3,3-Hepatafluorpropan und seine Mischungen mit Propan und/oder n-Butan und/oder i-Butan und/oder Dimethylether als Treibmittel bekannt. Arzneimittelsuspensionen oder die Verwendung der genannten Treibmittel in Arzneimittelsuspensionen sind in der EP-A-384 371 jedoch nicht beschrieben.

EP-A-315 783 erwähnt u.a. die Verwendung von 1,1,1,4,4,4-Hexafluorbutan als Treibgas sowie seine Eignung in Sprays für Asthmatiker. Jedoch ist das genannte Hexafluorbutan für sich als Treibgas ungeeignet, da es bei Normaldruck bei etwa 25 bis 30°C siedet und somit nicht den bei einem Treibgas erforderlichen Druck zur Ausbringung des Arzneimittels liefert. TG 227 hat demgegenüber unter Normaldruck einen Siedepunkt von -17,4°C und liefert daher bei den üblichen Anwendungstemperaturen den benötigten Druck von mehreren bar.

Aus einem Kongressbericht ,,Specialty fluoroaliphatic chemicals" by T.S. Johnson and H. Deger of the Hoechst Celanese Corporation, a paper presented at Chemspec USA, 1989, held on 10-11 October 1989 (ED1) wie auch aus einer Publikation "Blick auf Hoechst 1989, Nr. 8 (ED2)" wird berichtet, daß Hoechst an anderen Ersatzstoffen arbeitet, so an dem H-FKW 227, daß in der Kältetechnik und in medizinischen Aerosolen angewendet werden kann. Die Verwendung von H-FKW 222 für die Erzeugung von Inhalationsaerosolen ist dort nicht offenbart.

Aus Research Disclosure May 1989 (30161) wird berichtet, daß jetzt gefunden wurde, daß Kohlenwasserstoffe, wie Butan und Pentan; Kohlendioxid, Diemethylether, Stickstoff und Fluorkohlenwasserstoffe als Treibgase für Medikamente verwendet werden können, wobei diese Medikamente direkt in die Lauge appliziert werden. Die in diesem Abstract unter anderen genannte chemische Gruppe der unzähligen Fluorkohlenwasserstoffe ist so unpräzise, daß eine anwendbare technische Lehre nicht gegeben ist.

Wie nun gefunden wurde, ist TG 227 (1,1,1,2,3,3,3-Heptafluorpropan), gegebenenfalls in Mischung mit einem oder mehreren Treibgasen aus der Gruppe Propan, Butan, Pentan und DME (Dimethylether) therapeutisch anwendbaren Arneimittelsuspensionen besonders geeignet.

Die neben TG 227 verwendbaren Komponenten werden zugesetzt, wenn die Eigenschaften des Treibgases modifiziert werden sollen, z.B. wenn eine andere Dichte des verflüssigten Treibgases, ein anderer Druck oder ein anderes Löseverhalten eingestellt werden soll. Arzneimittelzubereitungen auf der Basis des neuen Treibgases enthalten einen Wirkstoff in feinverteilter Form, meist als Suspension, ferner im allgemeinen oberflächenaktive Stoffe, etwa ein Phospholipid (wie Lecithin), einen Ester eines Polyalkohols (wie Sorbit) mit höheren gesättigten oder ungesättigten Fettsäuren (z.B. Stearin-, Palmitin-, Ölsäure), beispielsweise Sorbitantrioleat, oder einen Polyethoxysorbitanester einer höheren, vorzugsweise ungesättigten Fettsäure, Der Hilfsstoff kann in der Mischung gelöst oder ungelöst vorliegen. Die mit dem neuen Treibgas hergestellten Suspensionen neigen z.T. zum Entmischen. Es hat sich jedoch gezeigt, daß solche entmischten Suspensionen durch Schütteln leicht wieder im Suspensionsmedium gleichmäßig verteilt werden können.

Die Mengenverhältnisse der einzelnen Mischungebestandteile des Treibgases (soweit TG 227 nicht allein verwendet wird) können in weiten Grenzen variert werden. Der Anteil (jeweils in Gewichteprozent) beträgt für TG 227 10 bis 100 %. Die Mischung kann darüber hinaus bis zu 50 % Propan und/oder Butan und/oder Pentan und/oder DME enthalten. Innerhalb der genannten Grenzen werden die Bestandteile so gewählt, daß sich insgesamt 100 % ergeben. Bevorzugt sind Treibgasmischungen, die 30 bis 100 % TG 227 enthalten.

Der Anteil des suspendierten Arzneistoffs an der fertigen Zubereitung beträgt zwischen 0,001 und 5 %, vorzugsweise 0,005 bis 3 %, insbesondere 0,01 bis 2 %. Die oberflächenaktiven Stoffe werden in Mengen von 0,01 bis 10 %, vorzugsweise 0,05 bis 5 %, insbesondere 0,1 bis 3 % zugegeben (hier wie bei den Arzneistoffen sind Gewichtsprozent der fertigen Zubereitung angegeben). Als Arzneistoffe in den neuen Zubereitungen können alle Substanzen dienen, die für die inhalative, ggf. auch für die intranasale Anwendung geeignet sind. Es handelt sich demnach insbesondere um Betamimetika, Anticholinergika, Steroide, Antiallergika, PAF-Antagonisten sowie Kombinationen aus solchen Wirkstoffen.

Im einzelnen seien als Beispiele genannt:
Als Betamimetika:
Bambuterol
Bitolterol
Carbuterol
Clenbuterol
Fenoterol
Hexoprenalin
Ibuterol
Pirbuterol
Procaterol
Reproterol
Salbutamol
Salmeterol
Sulfonterol
Terbutalin
Tulobuterol
1-(2-Fluor-4-hydroxyphenyl)-2-[4-(1-benzimidazolyl)-2-methyl-2-butylamino]ethanol
erythro-5'-Hydroxy-8'-(1-hydroxy-2-isopropylaminobutyl)-2H-1,4-benzoxazin-3-(4H)-on
1-(4-Amino-3-chlor-5-trifluormethylphenyl)-2-tert.-butyl-amino)ethanol
1-(4-Ethoxycarbonylamino-3-cyan-5-fluorphenyl)-2-(tert.-butylamino)ethanol.

Als Anticholinergika:
Ipratropiumbromid
Oxitropiumbromid
Trospiumchlorid
Benzilsäure-N-β-fluorethylnortropinestermethobromid

Als Steroide:
Budesonid
Beclometason (bzw. das 17, 21-Dipropionat)
Dexamethason-21-isonicotinat
Flunisolid

Als Antiallergika:
Dinatriumcromoglicat
Nedocromil

Als PAF-Antagonisten:
WEB 2086
WEB 2170
WEB 2347

Die Wirkstoffe können auch kombiniert werden, z.B. Betamimetika plus Anticholinergika oder Betamimetica plus Antiallergika.

Beispiele für Arzneimittelsuspensionen (Angabe in Gewichtsprozent):

| | | | |
|---|---|---|---|
| 1.) | 0,10 % Oxitropiumbromid | 2.) | 0,3 % Fenoterol |
| | 0,01 % Sojalecithin | | 0,1 % Sojalecithin |
| | 4,0 % Pentan | | 10,0 % Pentan |
| | 95,89 % TG 227 | | 70,0 % TG 227 |
| | | | 19,6 % TG 12 |
| | | | |
| 3.) | 0,1 % Ipratropiumbromid | 4.) | 0,3 % Fenoterol |
| | 0,1 % Sojalecithin | | 0,1 % Sojalecithin |
| | 20,0 % Propan | | 30,0 % TG 11 |
| | 20,0 % Butan | | 69,6 % TG 227 |
| | 49,8 % TG 11 | | |
| | | | |
| 5.) | 1,5 % Dinatrium | 6.) | 0,3 % Salbutamol |
| | cromoglicat | | 0,2 % Span 85 |
| | 0,1 % Tween 20 | | 20,0 % Pentan |
| | 98.4 % TG 227 | | 60,0 % TG 227 |
| | | | 19,5 % TG 12 |
| | | | |
| 7.) | 0,15 % Fenoterol | 8.) | 0,1 % Ipratropium- |
| | 0,06 % Ipratropium- | | bromid |
| | bromid | | 0,1 % Sojalecithin |
| | 0,10 % Sojalecithin | | 15,3 % Propan |
| | 40,00 % TG 11 | | 30,5 % TG 11 |
| | 19,69 % Propan | | 54,0 % TG 227 |
| | 40,00 % TG 227 | | |

## Patentansprüche

1. Arzneimittelsuspensionen in verflüssigten Treibgasen für die Erzeugung von Inhalationsaerosolen, gekennzeichnet durch einen Gehalt an 1,1,1,2,3,3,3-Heptafluorpropan als Treibmittel, gegebenenfalls in Mischung mit einem oder mehreren Treibgasen aus der Gruppe Propan, Butan, Pentan oder Dimethylether wobei der Gehalt an 1,1,1,2,3,3,3-Heptafluoropropan zwischen 10 und 100 Gew:-% bezogen auf das Treibmittel beträgt.

2. Arzneimittelsuspension nach Anspruch 1 dadurch gekennzeichnet, daß ein oberflächenaktiver Stoff aus der Gruppe der Phospholipide, Sorbitanester mit einer höheren gesättigten oder ungesättigten Fettsäure oder Polyethoxysorbitanester einer höheren, vorzugsweise ungesättigten Fettsäure zugesetzt ist.

3. Arzneimittelsuspension nach Anspruch 2, dadurch gekennzeichnet, daß der oberflächenaktive Stoff ein Lecithin, ein Polyoxyethylensorbitanoleat oder Sorbitantrioleat ist.

4. Arzneimittelsuspension nach Anspruch 1, 2 oder 3, dadurch gekennzeichnet, daß sie als Wirkstoff ein Betamimetikum ein Anticholinergikum, ein Steroid, ein Antiallergikum oder einen PAF-Antagonisten oder eine Kombination solcher Verbindungen enthält.

5. Arzneimittelsuspension nach Anspruch 4, dadurch gekennzeichnet, daß
als Betamimetikum
Bambuterol
Bitolterol
Carbuterol
Clenbuterol
Fenoterol
Hexoprenalin
Ibuterol
Pirbuterol
Procaterol
Reproterol
Salbutamol
Salmeterol
Sulfonterol
Terbutalin
Tulobuterol
1-(2-Fluor-4-hydroxyphenyl)-2-[4-(1-benzimidazolyl)-2-methyl-2-butylamino]ethanol
erythro-5'-Hydroxy-8'-(1-hydroxy-2-isopropylamino-butyl)-2H-1,4-benzoxazin-3-(4H)-on
1-(4-Amino-3-chlor-5-trifluormethylphenyl)-2-tert.-butyl- amino)ethanol
1-(4-Ethoxycarbonylamino-3-cyan-5-fluorphenyl)-2-(tert.-butylamino)ethanol;
als Anticholinergikum:
Ipratropiumbromid
Oxitropiumbromid
Trospiumchlorid
Benzilsäure-N-β-fluorethylnortropinestermethobromid;
als Steroid:
Budesonid
Beclometason (bzw. das 17, 21-Dipropionat)
Dexamethason-21-isonicotinat
Flunisolid;
als Antiallergikum:
Dinatriumcromoglicat
Nedocromil
als PAF-Antagonisten:
4-(2-Chlorphenyl)-9-methyl-2-[3-(4-morpholinyl)-3-propanon-1-yl]-6H-thieno[3,2-f][1,2,4]triazolo[4,3-a][1,4]diazepin
6-(2-Chlorphenyl)-8,9-dihydro-1-methyl-8-[4-morpholinyl)carbonyl]-4H-7H-cyclopenta[4,5]thieno[3,2-f][1,2,4]triazolo[4,3-a)[1,4]diazepin
6-(2-Chlorphenyl)-8,9,-dihydro-1-methyl-8-(N,N-dipropyl-aminocarbonyl)-4H-7H-cyclopenta[4,5]thieno[3,2-f][1,2,4]triazolo[4,3-a][1,4]diazepin
dient.

6. Arzneimittelsuspension nach Anspruch 4, dadurch gekennzeichnet, daß die Kombination der Wirkstoffe einer der in Anspruch 5 genannten Betamimetika und eines der in Anspruch 5 genannten Anticholinergika umfaßt.

7. Arzneimittelsuspension nach Anspruch 4, dadurch gekennzeichnet, daß die Kombination der Wirkstoffe eines der in Anspruch 5 genannten Betamimetika und Dinatriumcromoglicat umfaßt.

8. Arzneimittelsuspension nach Anspruch 4, dadurch gekennzeichnet, daß die Kombination der Wirkstoffe eines der in Anspruch 5 genannten Betamimetika und einen der in Anspruch 5 genannten PAF-Antagonisten enthält.

9. Arzneimittelsuspension nach Anspruch 4, dadurch gekennzeichnet, daß die Kombination der Wirkstoffe Dinatriumcromoglicat und einen der in Anspruch 5 genannten PAF-Antagonisten umfaßt.

10. Verwendung von 1,1,1 2'3,3'3-Heptafluorpropan als Treibmittel, gegebenenfalls in Mischung mit einem oder mehreren Treibgasen aus der Gruppe Propan, Butan, Pentan oder Dimethylether wobei der Gehalt an 1,1,1,2,3,3,3-Heptafluoropropan zwischen 10 und 100 Gew.-% bezogen auf das Treibmittel beträgt, bei der Herstellung von Arzneimittelsuspensionen für die Erzeugung von Inhalationsaerosolen.

## Claims

1. Pharmaceutical suspensions in liquefied propellant gases for the production of aerosols for inhalation, characterised in that they contain 1,1,1,2,3,3,3-heptafluoropropane as propellant, optionally in admixture with one or more propellant gases selected from the group comprising propane, butane, pentane or dimethyl ether, the content of 1,1,1,2,3,3,3-heptafluoropropane being between 10 and 100 % by weight, based on the propellant.

2. Pharmaceutical suspension according to claim 1, characterised in that a surfactant substance selected from the group comprising the phospholipids, sorbitan esters with a higher saturated or unsaturated fatty acid or polyethoxysorbitan esters of a higher, preferably unsaturated fatty acid is added.

3. Pharmaceutical suspension according to claim 2, characterised in that the surfactant substance is a lecithin, a polyoxyethylene sorbitan oleate or sorbitan trioleate.

4. Pharmaceutical suspension according to claim 1, 2 or 3, characterised in that it contains as active substance a beta-mimetic, an anticholinergic, a steroid, an antiallergic or a PAF antagonist or a combination of such compounds.

5. Pharmaceutical suspension according to claim 4, characterised in that the beta-mimetic used is:
bambuterol
bitolterol
carbuterol
clenbuterol
fenoterol
hexoprenaline
ibuterol
pirbuterol
procaterol
reproterol
salbutamol
salmeterol
sulfonterol
terbutalin
tulobuterol
1-(2-fluoro-4-hydroxyphenyl)-2-[4-(1-benzimidazolyl)-2-methyl-2-butylamino]ethanol
erythro-5'-hydroxy-8'-(1-hydroxy-2-isopropylamino-butyl)-2H-1,4-benzoxazin-3-(4H)-one
1-(4-amino-3-chloro-5-trifluoromethylphenyl)-2-tert.butyl-amino)ethanol
1-(4-ethoxycarbonylamino-3-cyano-5-fluorophenyl)-2-(tert.butylamino)ethanol;
the anticholinergic used is:
ipratropium bromide
oxitropium bromide
trospium chloride
benzylic acid-N-β-fluoroethylnortropinester
methobromide
the steroid used is:
budesonide
beclomethasone (or the 17,21-dipropionate)
dexamethasone-21-isonicotinate
flunisolide;
the antiallergic used is:
disodium cromoglycate
nedocromil
the PAF antagonists used are:
4-(2-chlorophenyl)-9-methyl-2-[3-(4-morpholinyl)-3-propanon-1-yl]-6H-thieno[3,2-f][1,2,4]triazolo[4,3-a][1,4]diazepine
6-(2-chlorophenyl)-8,9-dihydro-1-methyl-8-[4-morpholinyl)carbonyl]-4H-7H-cyclopenta[4,5]thieno[3,2-f][1,2,4]triazolo[4,3-a][1,4]diazepine
6-(2-chlorophenyl)-8,9-dihydro-1-methyl-8-[N,N-dipropylaminocarbonyl)-4H-7H-cyclopenta[4,5]thieno[3,2-f] [1,2,4]triazolo[4,3-a][1,4]diazepine.

6. Pharmaceutical suspension according to claim 4, characterised in that the combination of the active substances comprises one of the beta-mimetics mentioned in claim 5 and one of the anticholinergics mentioned in claim 5.

7. Pharmaceutical suspension according to claim 4, characterised in that the combination of the active substances comprises one of the beta-mimetics mentioned in claim 5 and disodium cromoglycate.

8. Pharmaceutical suspension according to claim 4, characterised in that the combination of the active substances comprises one of the beta-mimetics mentioned in claim 5 and one of the PAF antagonists mentioned in claim 5.

9. Pharmaceutical suspension according to claim 4, characterised in that the combination of the active substances comprises disodium cromoglycate and one of the PAF antagonists mentioned in claim 5.

10. Use of 1,1,1,2,3,3,3-heptafluoropropane as propellant, optionally in admixture with one or more propellant gases selected from the group comprising propane, butane, pentane or dimethyl ether, the content of 1,1,1,2,3,3,3-heptafluoropropane being between 10 and 100 % by weight, based on the propellant, in the preparation of pharmaceutical suspensions for producing aerosols for inhalation.

## Revendications

1. Suspensions médicamenteuses dans des gaz propulseurs liquéfiés pour la production d'aérosols pour inhalation, caractérisées par une teneur en 1,1,1,2,3,3,3-heptafluoropropane comme agent propulseur, éventuellement en mélange avec un ou plusieurs gaz propulseurs du groupe du propane, du butane, du pentane ou du diméthyléther où la teneur en 1,1,1,2,3,3,3-heptafluoropropane est située entre 10 et 100% en masse par rapport à l'agent propulseur.

2. Suspension médicamenteuse selon la revendication 1 caractérisée en ce qu'une substance tensioactive du groupe des phospholipides, des esters du sorbitan avec un acide gras supérieur saturé ou insaturé ou des esters de polyéthoxysorbitan et d'un acide gras supérieur, de préférence insaturé est ajoutée.

3. Suspension médicamenteuse selon la revendication 2 caractérisée en ce que la substance tensioactive est une lécithine, un oléate de polyoxyéthylènesorbitan ou le trioléate de sorbitan.

4. Suspension médicamenteuse selon la revendication 1, 2 ou 3 caractérisée en ce qu'elle contient comme principe actif un bêtamimétique, un anticholinergique, un stéroïde, un antiallergique ou un antagoniste du PAF ou une combinaison de tels composés.

5. Suspension médicamenteuse selon la revendication 4 caractérisée en ce que le bêtamimétique est:
le bambutérol,
le bitoltérol,
le carbutérol,
le clenbutérol,
le fénotérol,
l'hexoprénaline,
l'ibutérol,
le pirbutérol,
le procatérol,
le reprotérol,
le salbutamol,
le salmétérol,
le sulfontérol,
la terbutaline,
le tulobutérol,
le 1-(2-fluoro-4-hydroxyphényl)-2-[4-(1-benzimidazolyl)-2-méthyl-2-butylamino]éthanol,
l'érythro-5'-hydroxy-8'-(1-hydroxy-2-isopropylaminobutyl)-2H-1,4-benzoxazin-3-(4H)-one,
le 1-(4-amino-3-chloro-5-trifluorométhylphényl)-2-tert.butylamino)éthanol,
le 1-(4-éthoxycarbonylamino-3-cyano-5-fluorophényl)-2-(tert.butylamino)éthanol,
l'anticholinergique est:
le bromure d'ipratropium,
le bromure d'oxitropium,
le chlorure de trospium,
le méthobromure d'ester d'acide benzilique et de N-β-fluoroéthylnortropine,
le stéroïde est:
le budesonide,
la beclométasone (ou le 17,21-dipropionate),
le 21-isonicotinate de dexaméthasone,
le flunisolide,
l'antiallergique est:
le cromoglicate de disodium,
le nédocromil,
l'antagoniste du PAF est:
la 4-(2-chlorophényl)-9-méthyl-2-[3-(4-morpholinyl)-3-propanon-1-yl]-6H-thiéno[3,2-f][1,2,4]triazolo[4,3-a][1,4]diazépine,
la 6-(2-chlorophényl)-8,9-dihydro-1-méthyl-8-[4-morpholinyl)carbonyl]-4H,7H-cyclopenta[4,5]thiéno[3,2-f][1,2,4]triazolo[4,3-a][1,4]diazépine,
la 6-(2-chlorophényl)-8,9-dihydro-1-méthyl-8-(N,N-dipropylaminocarbonyl)-4H,7H-cyclopenta[4,5]thiéno[3,2-f][1,2,4]triazolo[4,3-a][1,4]diazépine.

6. Suspension médicamenteuse selon la revendication 4 caractérisée en ce que la combinaison des principes actifs comprend l'un des bêtamimétiques cités dans la revendication 5 et l'un des anticholinergiques cités dans la revendication 5.

7. Suspension médicamenteuse selon la revendication 4 caractérisée en ce que la combinaison des principes actifs comprend l'un des bêtamimétiques cités dans la revendication 5 et du cromoglicate de disodium.

8. Suspension médicamenteuse selon la revendication 4 caractérisée en ce que la combinaison des principes actifs comprend l'un des bêtamimétiques cités dans la revendication 5 et l'un des antagonistes du PAF cités dans la revendication 5.

9. Suspension médicamenteuse selon la revendication 4 caractérisée en ce que la combinaison des principes actifs comprend du cromoglicate de disodium et l'un des antagonistes du PAF cités dans la revendication 5.

10. Utilisation du 1,1,1,2,3,3,3-heptafluoropropane comme agent propulseur, éventuellement en mélange avec un ou plusieurs gaz propulseurs du groupe du propane, du butane, du pentane et du diméthyléther où la teneur en 1,1,1,2,3,3,3-heptafluoropropane est située entre 10 et 100% en masse par rapport à l'agent propulseur, dans la préparation de suspensions médicamenteuses pour la production d'aérosols pour inhalation.
